# EUROPEAN PATENT SPECIFICATION

(11) **EP 3 399 310 B1**
(45) Date of publication and mention of the grant of the patent: **24.11.2021**
(21) Application number: 15912110.2
(22) Date of filing: 28.12.2015
(51) Int. Cl.: G01N 33/493, G01N 27/06, A61B 10/00

(54) **HEALTH MONITORING SYSTEM, HEALTH MONITORING METHOD, AND HEALTH MONITORING PROGRAM**
GESUNDHEITSÜBERWACHUNGSSYSTEM, GESUNDHEITSÜBERWACHUNGSVERFAHREN UND GESUNDHEITSÜBERWACHUNGSPROGRAMM
SYSTÈME DE SURVEILLANCE DE SANTÉ, PROCÉDÉ DE SURVEILLANCE DE SANTÉ ET PROGRAMME DE SURVEILLANCE DE SANTÉ

(43) Date of publication of application: 07.11.2018
(73) Proprietor: Symax Inc., Tokyo 125-0031 (JP)
(72) Inventor: TSURUOKA, Maria, Tokyo 125-0031 (JP); WADA, Yoshitaka, Tokyo 125-0031 (JP)
(74) Representative: Epping - Hermann - Fischer
(86) International application number: PCT/JP2015/086575
(87) International publication number: WO 2017/115444

(56) References cited:
- WO-A1-2010/002097
- WO-A1-2012/105748
- JP-A- H 085 631
- JP-A- H07 102 610
- JP-A- 2000 241 411
- JP-A- 2001 265 822
- JP-A- 2003 302 397
- JP-A- 2004 279 219
- JP-A- 2005 172 647
- JP-A- 2006 225 966
- JP-A- 2009 258 078

## Description

### BACKGROUND

### 1. Technical Field

This invention relates to a health monitoring system, a health monitoring method, and a health monitoring program. This invention particularly relates to a health monitoring system to be installed on a toilet, a health monitoring method, and a health monitoring program for analyzing excreted urine and estimating a disease.

### 2. Description of Related Art

In response to rising health consciousness of recent years, there have been many conventional services of assaying the condition of urine (amount or component), monitoring a health condition, and giving advice. In the presence of abnormality in a body, the condition of urine changes easily. Hence, monitoring the condition of urine on a routine basis is effective in detecting abnormality in a body.

For example, Japanese Patent Application Publication No. 2013-36817 (JP 2013-36817 A) discloses an excreted urine information measurement device relating to the aforementioned technique of assaying urine. This measurement device stores data indicating a correlation between the concentration of a specific component in one excretion of urine from a subject of measurement and the concentration of the specific component in total urine excreted throughout the day of the measurement. This measurement device performs a conversion calculation to acquire the concentration of the specific component in the total urine excreted through the day from the subject of the measurement using the correlation, and calculates the amount of the excreted specific component in the total urine excreted throughout the day from the subject of the measurement using the acquired concentration.

Japanese Patent Application Publication No. 2013-90748 (JP 2013-90748 A) discloses an excreted urine information measurement device that calculates the amount of excreted urine and a urine flow rate by using a toilet bowl storing urine, and urine data measurement device that measures the volume or weight of urine stored in the bowl. The excreted urine measurement device described in JP 2013-90748 A calculates the amount of excreted urine and a urine flow rate based on a water level at a time of start or finish of urination or a change rate of a water level between the time of start and the time of finish of the urination, applies a given vibration model to the calculated data, and processes the calculated data through a particle filter, thereby calculating excreted urine information.

JP 2003 302397 A discloses an excreted urine information measurement device that estimates the concentration of specific components of the excreted urine and the absolute quantity of the specific component in the excreted urine. The calculations are based on measuring changes in temperature of the toilet device, the stored-water and the water/urine mixture and using a biological-information measuring device.

### SUMMARY

The invention described in JP 2013-36817 A is roughly formed of a housing and a sensor unit. This requires grasping of the housing with a hand of a subject of measurement, etc., and spattering of urine excreted by the subject of measurement over the sensor unit. Thus, sufficient usability has not always been achieved.

Regarding the invention described in JP 2013-90748 A, means for measuring the volume or weight of urine stored in the toilet bowl uses an element forming a toilet such as water level data about a puddle of water in the bowl or measurement of the pressure of sewage water in a sewage pipe. This has made it impossible to apply the invention described in JP 2013-90748 A to an existing toilet. Hence, the excreted urine information measurement device described in JP 2013-90748 A is poor in versatility and sufficient usability and has not always been achieved by this measurement device.

A disadvantage of the device disclosed in JP 2003 302397 A is that a measurement of a specific component requires the taking of a urine sample by the urine-sampling unit which is not a part of a standard toilet device. The collection of a urine sample in a separate vessel and its associated problems reduces the user friendliness of the system.

It is therefore an object of this invention to provide a simple and easy-to-use health monitoring system, a health monitoring method, and a health monitoring program to be employed for analysis of excreted urine including such as assay of a urine component and estimation of a disease based on a result of the analysis.

A health monitoring system according to this invention is defined in claim 1.

The health monitoring system according to the invention may further comprise features as set forth in claims 2-8.

A health monitoring method according to this invention is set forth in claim 9.

A health monitoring program according to this invention is set forth in claim 10. A urine component can be measured just by installing the health monitoring system according to this invention on an existing toilet and just through normal excretion by a subject of the measurement. This achieves more simple and more hygienic measurement than measurement to be made by spattering urine over a device, thereby enhancing usability.

The health monitoring system and the health monitoring method according to this invention analyze a urine amount by analyzing the motion of a fluid by means of a fluid simulation. This makes it possible to take a degree of dilution of excreted urine with a puddle of water into consideration, so that the excreted urine can be assayed with high accuracy.

### <Advantageous Effects>

The health monitoring system and the health monitoring method according to this invention are capable of analyzing excreted urine information and estimating a disease more easily with enhanced usability.

### BRIEF DESCRIPTION OF THE DRAWINGS

Features, advantages, and technical and industrial significance of exemplary embodiments of the invention will be described below with reference to the accompanying drawings, in which like numerals denote like elements, and wherein:
Fig. 1 is a system diagram showing an exemplary configuration of a health monitoring system;
Fig. 2 is a block diagram showing an exemplary configuration of the health monitoring system 500 according to an embodiment;
Fig. 3 schematically shows an exemplary outline of the health monitoring system 500 according to the embodiment;
Fig. 4 schematically shows an exemplary outline of a measurement device 200 of the health monitoring system 500;
Fig. 5 schematically shows an exemplary internal configuration of a measurement unit 210 forming the measurement device 200 according to a first embodiment;
Fig. 6 schematically shows an exemplary internal configuration of the measurement unit 210 forming the measurement device 200 according to a second embodiment;
Fig. 7 schematically shows an exemplary configuration of a film forming an imaging part 212 and that of a reagent;
Fig. 8 is a data conceptual view showing an exemplary data configuration of a database for data stored in the health monitoring system 500;
Fig. 9 is a flowchart showing an exemplary process performed by the health monitoring system 500; and
Fig. 10 an exemplary data configuration associating measurement/analysis results of the health monitoring system 500 with information such as diseases.

### DETAILED DESCRIPTION OF EMBODIMENTS

An embodiment of the present invention will be described next based on the drawings.

### <Overview>

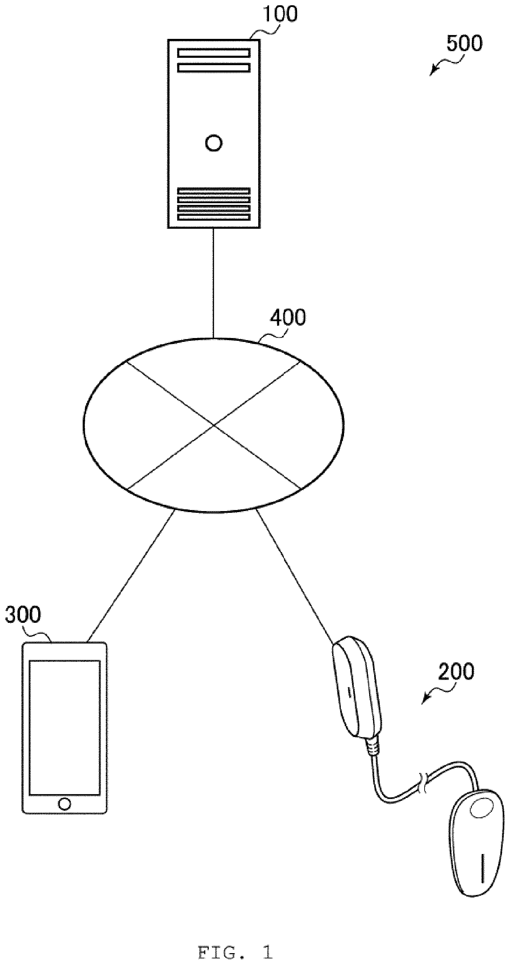

Fig. 1 is a system diagram showing an exemplary configuration of a health monitoring system according to a first embodiment of this invention.

As shown in Fig. 1, this system includes a server 100, a measurement device 200, and a user terminal 300. The server 100 is connected to the measurement device 200 and the user terminal 300 via a network 400. For simplification of the illustration, Fig. 1 shows only one server 100, one measurement device 200, and one user terminal 300. However, two or more servers 100, two or more measurement devices 200, and two or more user terminals 300 can certainly be provided. A specific device of the user terminal 300 is not limited to a smartphone illustrated in the drawing. For example, the user terminal 300 may be a mobile terminal, a tablet terminal, a personal computer, or a different electronic device. Additionally, this system may use a cloud service (including both a public cloud and a private cloud). Alternatively, a service may be provided by a common or dedicated server installed physically in a target facility.

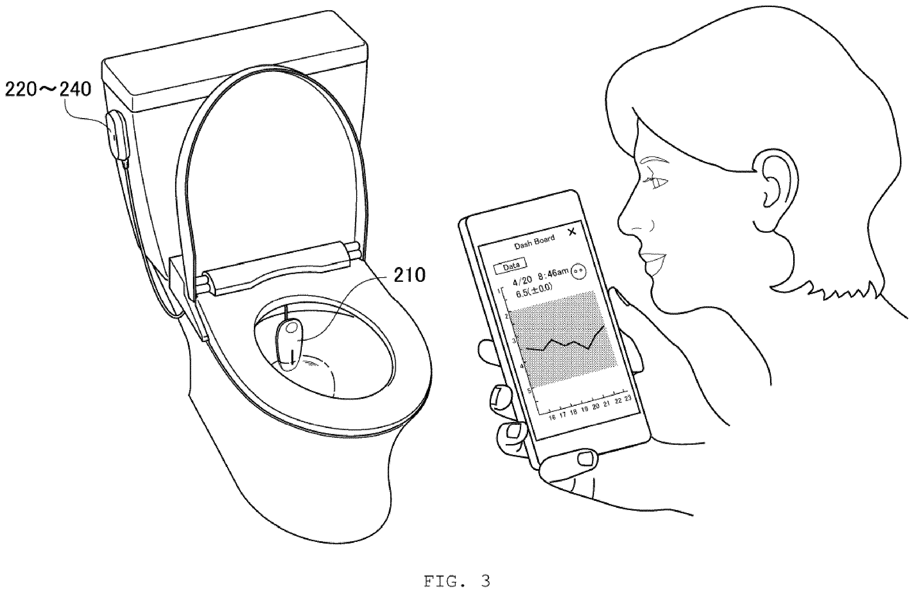

The user terminal 300 has an application (hereinafter called a "health monitoring application") forming a part of the health monitoring system according to the embodiment of the present invention. This application is for displaying a monitoring result about a health condition (including an assay result and an estimation result). As shown in Fig. 3, a user is allowed to check the health condition of the user himself or herself by viewing a display provided by the health monitoring application.

As shown as an example in Fig. 3, in the health monitoring system 500, the measurement device 200 is installed for example on an existing toilet, the measurement device 200 measures fluid information about a fluid in a puddle of water into which urine excreted by a user of the toilet has flowed, and the server 100 analyzes the excreted urine by analyzing a fluid model formed by modeling a region where the fluid flows based on the measured fluid information. Then, a disease of the user can be estimated based on excreted urine information about the analyzed excreted urine. This allows the health monitoring 500 to determine a symptom of a disease, or positive or negative while a user is only required to urinate normally at home or at a workplace. In this way, a simple, easy-to-use, and high-sustainability health monitoring service can be provided.

The applicability of the health monitoring system 500 is not limited to homes or workplaces but the health monitoring system 500 is usable for health management of patients in care facilities or hospitals, thereby contributing to risk reduction on a management side. The "excreted urine information" mentioned herein means various types of information about urine excreted by a user and may contain the amount and temperature of excreted urine, a component in the excreted urine, etc.

The example shown in the present embodiment employs a cloud computing system. However, this is not the only example but the health monitoring system 500 may be formed of the measurement device 200 alone or by the measurement device 200 and the user terminal 300. This example is about a service using a cloud service. This example is further applicable to a service using artificial intelligence (machine learning by means of deep leaning, for example) such as a cloud doctor service (a service of diagnosing the health condition or physical condition of a patient via a network, for example) or a cloud mother service (a service or monitoring the health or physical condition of a child via a network, for example).

### <Configuration>

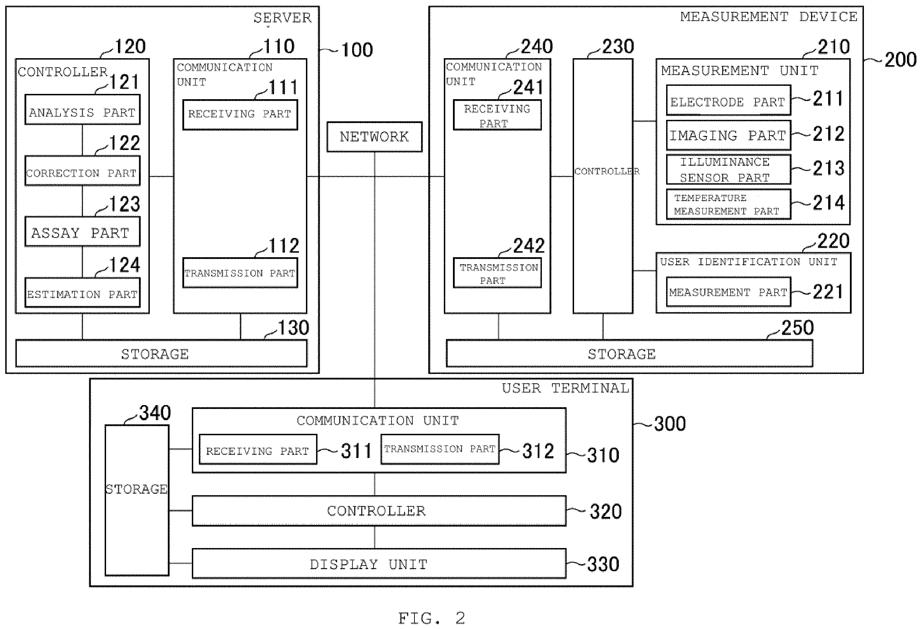

The respective configurations of the server 100, the measurement device 200, and the user terminal 300 will be described below. Fig. 2 is a block diagram showing an exemplary functional configuration of the server 100, that of the measurement device 200, and that of the user terminal 300. Where appropriate, the arrangement of each part can be changed between the server 100, the measurement device 200, and the user terminal 300 in response to the operating environment or situation of each unit, for example. For example, the analysis part 121, a correction part 122, an assay part 123, and the estimation part 124 of the server 100 may be arranged in a controller 230 of the measurement device 200 or in a controller 320 of the user terminal 200. As shown in Fig. 2, the server 100 includes a communication unit 110, a controller 120, and a storage 130.

The server 100 can be configured in multiple stages. For example, the server 100 may be formed of a server (relay server) installed in a facility and a server covering a particular area including a plurality of facilities or covering every area. The following describes examples of timing of transmission from the relay server: (1) cyclically (at regular time intervals determined in consideration of the capacity of the storage 130, for example); and (3) at a time when a threshold set for the storage capacity of a storage 250 is reached.

The communication unit 110 includes a receiving part 111 and a transmission part 112. The communication unit 110 has the function of making communication with the measurement device 200 and the user terminal 300 via the network 400. This communication can be either wire communication or wireless communication. Further, any communication protocol can be used as long as mutual communication can be established.

The receiving part 111 has the function of receiving measurement data, etc. from each measurement device 200 and each user terminal 300 via the network 400 under control by the controller 120, and transmitting the received measurement data to the controller 120. More specifically, the receiving part 111 receives the following from the measurement device 200: water temperature information about a puddle of water in a bowl of a toilet and water temperature information about water containing the puddle of water and urine excreted by a user of the toilet (hereinafter called "water containing excreted urine"); voltage information resulting from a potential difference between electrodes observed by dipping the electrodes in the water containing excreted urine; user identification information for identifying the user; illuminance information; and imaging information obtained by making an imaging part 212 capture an image of a film having reacted with a reagent (hereinafter called "imaging information"). The receiving part 111 transmits the received information to the controller 120.

The transmission part 112 has the function of transmitting control data, etc. to each measurement device 200, and transmitting monitoring result data, etc. to each user terminal 300 via the network 400 under control by the controller 120. More specifically, the transmission part 112 transmits the following to the measurement device 200: user information (ID information, for example) to be stored in the storage 130 for control over a user identification unit 220; and dynamic parameter data, etc. required for measurement and image capturing by the measurement unit 210 and identification by the user identification unit 220, for example. The transmission part 112 further transmits display data to the user terminal 300 indicating an assay result about an assayed urine component and a monitoring result such as an estimation result indicating whether being positive or negative for an estimated disease.

The controller 120 is a processor including the analysis part 121, the correction part 122, the assay part 123, and the estimation part 124, and having the function of controlling each part of the server 100. In response to receipt of an assay result transmitted from the assay part 123 or receipt of an estimation result transmitted from the estimation part 124, the controller 120 generates display data for display of the received result in a text, table, or graph format on a display unit 330 of the user terminal 300. To transmit the generated display data to the user terminal 300, the controller 120 transmits this data to the transmission part 112.

The analysis part 121 has the function of analyzing excreted urine by analyzing a fluid model formed by modeling a region where a fluid flows based on fluid information. The "fluid information" mentioned herein means information required for fluid analysis. The fluid information contains shape information about a toilet bowl (hereinafter called "shape information"), and water amount information and water temperature information about a puddle of water in the toilet bowl, for example.

More specifically, based on at least one of pieces of information including shape information about a toilet bowl, and water amount information and water temperature information about a puddle of water in the toilet bowl, for example, and based on a fluid model formed by modeling a fluid flowing around the measurement unit 210, the analysis part 121 analyzes the fluid around the measurement unit 210 to calculate a urine amount, thereby analyzing excreted urine. The analysis part 121 may use at least one information about toilet environment such as amount information for example about a cleaner or component information about the cleaner, etc., in addition to the shape information about the toilet bowl, and the water amount information and the water temperature information about the puddle of water in the toilet bowl, and analyze excreted urine information for example by modeling a fluid based on these pieces of information. This eliminates the need to extract only excreted urine for measurement of the amount of the urine or measure a urine amount based on a change rate of a water level using a measurement instrument, etc. attached to a toilet bowl or a drain pipe. In this way, an easy-to-use health monitoring system can be provided to a user.

The aforementioned modeling of a fluid may be used for assay made by building a prediction model showing how the water temperature of a puddle of water and that of water containing excreted urine change to finally converge using regression analysis by means of an SVM (support vector machine), for example, and based on water temperature information generated as a result of measurement of the water temperature of the puddle of water and that of the water containing excreted urine. This regression analysis may be made by combining the SVM and a data configuration derived by a Kernel method. As another example, assay may be made by building a regression model by means of regression analysis using an MCMC (Markov chain Monte Carlo) method. As another example of modeling a fluid region by means of a fluid simulation, a finite-element method or a CFD (computational fluid dynamics) method may be further used.

The correction part 122 has the function of correcting voltage information based on water amount information and excreted urine information containing a urine amount. More specifically, the correction part 122 calculates a degree of dilution by dividing a urine amount by the sum of a water amount and the urine amount to correct voltage information based on the calculated degree of dilution, for example. This makes it possible to acquire voltage information while taking dilution for example with a puddle of water in a toile bowl into consideration, and eventually, assay a urine component.

The correction part 122 has the function of correcting imaging information based on illuminance information. The "illuminance information" mentioned herein means information indicating the illuminance (brightness) (lx) of a film surface of the imaging part 212. More specifically, the correction part 122 corrects the imaging information by adjusting the lightness of an RGB value at a proper value based on the illuminance information, for example. This makes it possible to acquire an RGB value while taking influence by illumination into consideration, so that color can be measured with high accuracy.

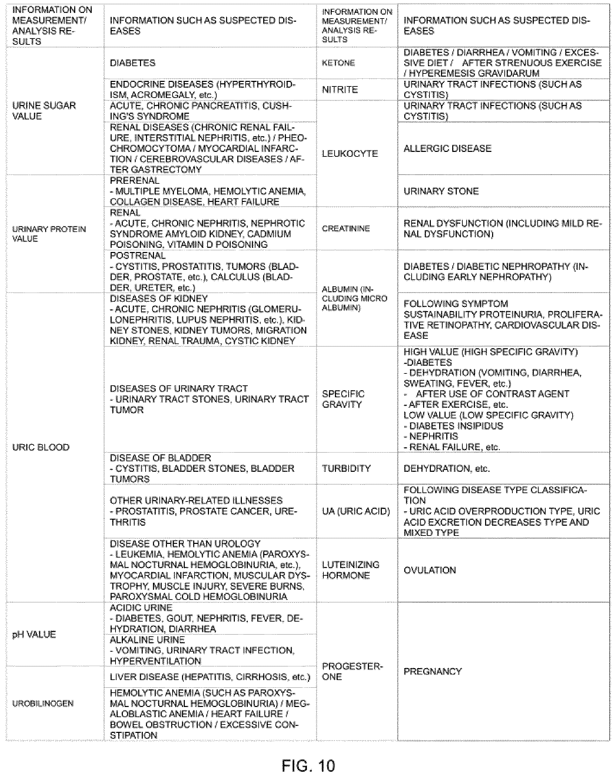

The assay part 123 has the function of assaying a urine component based on voltage information or corrected voltage information (hereinafter called "voltage information (as corrected)"). More specifically, the assay part 123 assays the concentration of molecule of a component in urine such as chloride, glucose, potassium, sodium, or urea, for example, based on the voltage information (as corrected). As shown in Fig. 10, the assay part 123 can also assay a pH value. By doing so, the assay part 123 can make assay with high accuracy even if excreted urine is diluted with a puddle of water. Further, to generate display data including a result of this assay on the user terminal 300, the assay part 123 transmits this result to the controller 120.

The assay part 123 further has the function of assaying a urine component based on imaging information or corrected imaging information (hereinafter called "imaging information (as corrected)"). More specifically, the assay part 123 measures the color of chromogenic reaction of a specific component in urine with a reagent based on imaging information (an RGB value) to assay the specific component corresponding to the measured color in the urine or the concentration of the specific component, for example. Further, to generate display data including a result of this assay on the user terminal 300, the assay part 123 transmits this result to the controller 120. Thus, assay of a specific component in urine and assay of the concentration of the specific component do not require visual check by a person, etc. but can be made by bioassay (using an immunochromato method, for example) automatically and simply without intervention by a person.

The estimation part 124 has the function of estimating a disease of a user based on excreted urine information about analyzed excreted urine. More specifically, for example, the estimation part 124 estimates a disease of a user based on an assayed specific component in urine (more specifically, the concentration of the specific component, for example). As shown in Fig. 10, for example, the estimation part 124 calculates a glucose level in urine by assaying the concentration of glucose in the urine to estimate whether the user is positive or negative for diabetes. Fig. 10 shows an example of association between a result of different measurement by the measurement unit 210 or a result of different assay by the assay part 123 (called a "result of measurement and assay") and information for example about a disease estimated based on the result of measurement and assay. Estimation by the estimation part 124 may include the estimation given in the example of the association. To generate display data including a result of this estimation on the user terminal 300, the estimation part 124 transmits this result to the controller 120.

The estimation part 124 can make estimation by the following ways: (1) estimation using a threshold; and (2) estimation by machine learning. As an example of the estimation (1), the estimation part 124 compares a measurement result and a threshold stored in the storage 130 to determine normality (or being negative) if the measurement result falls below the threshold and determine abnormality (or being positive) if the measurement result exceeds the threshold, for example, thereby estimating a disease. For the estimation (2), the estimation part 124 extracts a feature quantity from a measurement result and generates a feature vector based on the extracted feature quantity. The generated feature vector is identified based on dictionary data (data generated by using a set of a measured value and a test result linked with this measured value (a result indicating whether being positive or negative, etc. for a disease obtained based on an assay result and an estimation result) used in a plurality of cases, and functioning as training data (teacher data) for machine learning). Based on a result of this identification, the estimation part 124 estimates a disease. A neutral network (perceptron), an SVM, etc. can be used as techniques of this machine learning. Thus, as a result of learning effect of the machine learning, accuracy of estimation by the estimation part 124 can be increased.

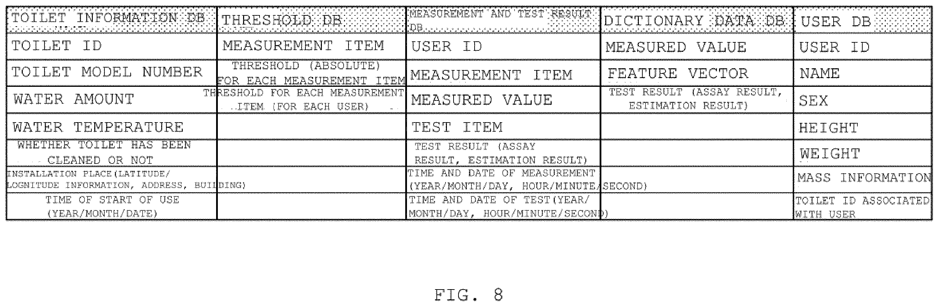

The storage 130 has the function of storing various programs, various types of data, and various parameters required for operating the server 100. More specifically, the storage 130 stores fluid information (shape information about a bowl of a toilet and water amount information about a puddle of water in the toilet), imaging information, weight information, illuminance information, user identification information, and parameters required for the operations of the communication unit 110, the controller 120, and the storage 130, for example. As shown in Fig. 8, for example, the storage 130 saves and stores information required for analysis, assay, etc., measurement results, and test results (assay results and estimation results) into various databases (hereinafter called "DBs").

The DB is not the only method of storing and managing data. The data may also be saved and stored in every type of configuration file such as a definition file, a parameter file, and a temporary file (hereinafter called a "configuration file"). The storage 250 is typically realized by every type of recording medium such as an HDD (hard disc drive), an SSD (solid state drive), and a flash memory (an SD (secure digital) memory card), for example. Various DBs are described later in the section <Data>. The configuration of the server 100 is as described above.

The configuration of the measurement device 200 will be described next.

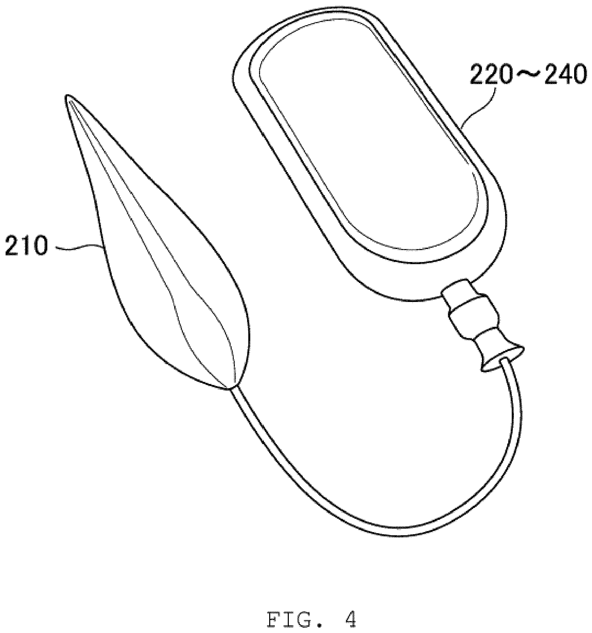

As shown in Fig. 2, the measurement device 200 includes the measurement unit 210, the user identification unit 220, the controller 230, a communication unit 240, and the storage 250. All the units of the measurement device 200 can be arranged in a plurality of devices. As shown in Fig. 4, for example, the measurement unit 210 may be arranged in a device shown in the right side of Fig. 4, whereas the user identification unit 220, the controller 230, the communication unit 240, and the storage 250 may be arranged together in a different device shown in the left side of Fig. 4. By doing so, the device only including the measurement unit 210 can be installed for example in a toilet bowl, whereas the different device can be installed in a range that does not cause problem in terms of communication. In this way, the arrangement of the devices is allowed to have compatibility with the shape of a toilet.

The measurement unit 210 includes an electrode part 211, the imaging part 212, an illuminance sensor part 213, and a temperature measurement part 214. As shown in Fig, 3, for example, the measurement unit 210 may be installed in such a manner that the electrode part 211, the imaging part 212, and the temperature measurement part 214 are dipped at least partially in a puddle of water in a toilet bowl. If the measurement unit 210 receives transmission of input of start of measurement given by a user through input means of the controller 230, the measurement unit 210 can make the electrode part 211, the imaging part 212, the illuminance sensor part 213, and the temperature measurement part 214 start corresponding measurement using the transmission as a trigger.

If at least one of temperature information generated by the electrode part 211 (the water temperature of a puddle of water or that of water containing excreted urine, for example) and voltage information generated by the temperature measurement part 214 (a potential different, for example) reaches a predetermined threshold, the measurement unit 210 can make each part forming the measurement unit 210 start or finish corresponding measurement automatically. This makes it possible to start measurement during normal urination of a user without requiring the user to select start or finish measurement each time the measurement is to be started or finished. In this way, an easy-to-use measurement device can be provided. It is preferable the threshold value for the temperature information for the start of the measurement be set as 38°C.

If the user identification unit 220 completes identification of a user, the measurement unit 210 may start measurement automatically using this completion as a trigger. Additionally, a threshold may be provided for each measurement item. If data reaching this threshold is acquired, the measurement unit 210 may finish measurement using this acquisition as a trigger. Alternatively, the measurement unit 210 may start or finish measurement in response to operational input given manually through the display unit 330 of the user terminal 300. Still alternatively, a human sensor (not shown in the drawings) may be provided to the measurement device 200. If a sign of a person is detected for example with an infrared ray from the human sensor, the measurement unit 210 may start measurement using this detection as a trigger. Further, if the sign of the person disappears, the measurement unit 210 may finish the measurement using this disappearance as a trigger.

The electrode part 211 has the function of measuring electromotive force (a potential difference or voltage value) generated by an electrolyte that is a specific component in urine and measuring the value of a current flowing between electrodes dipped in water containing excreted urine using two or more electrodes, thereby generating voltage information. More specifically, for measuring the concentration of the specific component in urine, the electrode part 211 is formed of two or more electrodes, a potentiometer, and an ammeter, for example. The electrode part 211 uses one electrode as a reference electrode and a different electrode as a working electrode, for example. Then, these electrodes are dipped in water containing excreted urine to measure a difference in electromotive force between the working electrode responsive to the concentration (activity) of a urine component in the water containing excreted urine targeted for assay and the reference electrode with the potentiometer. Based on a result of the measurement, the electrode part 211 generates voltage information. To transmit the generated voltage information to the server 100, the electrode part 211 transmits this voltage information to a transmission part 242 via the controller 230.

The "voltage information" mentioned herein means information about electromotive force (a potential difference or a voltage value) generated by a specific component (an electrolyte) in urine using the electrodes of the electrode part 211. The example mentioned herein uses an ion-selective electrode method. Alternatively, a GOD (glucose oxidase) method may be used. Still alternatively, a three-electrode method may be used by adding an electrode to function as a counter electrode. These methods achieve measurement for example of the concentration of the specific component in urine based on the generated voltage information.

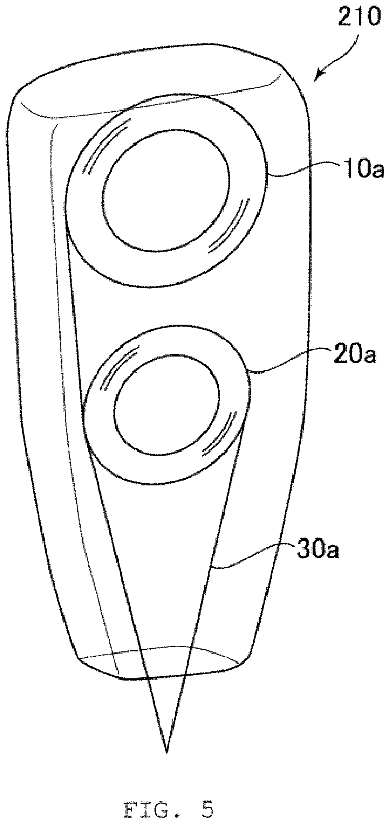

The imaging part 212 has the function of making a specific component in urine produce color reaction with a reagent, etc. using bioassay and capturing an image of the reaction. More specifically, the imaging part 212 is formed of a film to change in color in response to a component in water containing excreted urine, and imaging means that captures an image of the film, for example. The "film" mentioned herein can be made of any material as long as the film permits addition of a reagent, can make a specific component in urine produce color reaction with the reagent, and can be formed into a tape-like shape (a thin band-like shape capable of being wound up on a reel, for example). The film may be made of a polymer component such as synthetic resin or made of a fibrous material such as paper or cloth. Preferably, the film is transparent. If an immunochromato method is used as an assay method, for example, the configuration of the imaging part 212 includes a sample pad, a conjugate pad, a test line (detection line), a control line, a membrane, an absorption pad, etc. However, this is not the only configuration of the imaging part 212. The configuration of the film will be described later using Figs 5, 6, and 7.

The sample pad is dipped in water containing excreted urine to absorb the water containing excreted urine. An RGB (red, green, blue) value of color appearing as a result of color reaction at the test line and the control line is read by capturing an image of the color using the imaging means such as a camera. To transmit information about the imaging (read RGB value) to the server 100, the imaging part 212 transmits this information to the transmission part 242 via the controller 230.

The server 100 measures the color appearing as a result of the color reaction based on the transmitted imaging information. This achieves color measurement at lower cost than a method of reading a wavelength, etc. using a spectroscope, for example.

Unfortunately, the conventional techniques described in JP 2013-36817 A and JP 2013-90748 A are inapplicable to a test method such as an immunochromato assay method using antigen-antibody reaction by which a complex is formed by adding an analyte to a pad and generating antigen-antibody reaction, the complex is combined with a different type of antibody to generate a different complex, and pregnancy or whether being positive or negative for a disease is determined based on reaction (such as appearance of color) of the combination.

The health monitoring system according to this invention further includes the imaging part 212 with the film to change in color in response to a component in a puddle of water into which excreted urine has flowed, and the imaging means that generates imaging information by capturing an image of the film. The correction part corrects the imaging information based on water amount information and excreted urine information containing the amount of excreted urine. The assay part 123 assays a urine component based on the corrected imaging information. Thus, the health monitoring system of this disclosure is further applicable to a test method such as an immunochromato assay method using antigen-antibody reaction and achieves more measurements than a conventional measurement device for excreted urine information to be installed on a toilet, for example.

The illuminance sensor part 213 has the function of measuring the illuminance (brightness) of a surface of the film as a target of image capturing by the imaging part 212. The illuminance sensor part 213 is formed of a light-receiving element such as a photodiode. For example, the illuminance sensor part 213 converts light incident on the light-receiving element to a current to detect illuminance. To transmit information about the detected illuminance, the illuminance sensor part 213 transmits the illuminance information to the transmission part 242 via the controller 230. The server 100 can correct a result of the aforementioned color measurement using the transmitted illuminance information. This makes it possible to obtain a result of the color measurement while taking illuminance determined by illumination into consideration, so that reaction of a specific component in urine targeted for assay can be assayed with high accuracy.

The temperature measurement part 214 has the function of measuring the temperature of a puddle of water in a toilet bowl or the temperature of water containing excreted urine to generate water temperature information. The temperature measurement part 214 is formed of a thermistor, an oscillator, and a counter, for example. The thermistor outputs change in a resistance value resulting from temperature change. The oscillator converts this change in a resistance value to a frequency. The counter measures this frequency to measure temperature. To transmit the water temperature information to the server 100, the temperature measurement part 214 transmits this information to the transmission part 242 via the controller 230.

The user identification unit 220 has the function of identifying a user as a target of monitoring by the health monitoring system 500 using a toilet. As shown in Fig. 3, for example, the user identification unit 220 is connected to the measurement unit 210 via a wire such as a cable. To install the user identification unit 220 on a tank storing cleaning water, the user identification unit 220 may include means of sticking the user identification unit 220 to a ceramic unit such as the tank. The user identification unit 220 may include different type of attachment means.

More specifically, the user identification unit 220 for example reads information (a QR code (registered trademark)) for uniquely identifying a user output from the health monitoring application installed on the terminal 300 belonging to the user (this information for identifying the user is hereinafter called "user identification information"), magnetic information for uniquely identifying the user of an IC (integrated circuit) card belonging to this user, or information (receiving signal intensity information or radio wave receiving intensity information, for example) for uniquely identifying the user of a wireless LAN (local area network) such as WiMAX (Worldwide Interoperability for Microwave Access), WiFi (Wireless Fidelity), or Bluetooth (registered trademark), thereby identifying the user.

As described above, a user can be identified automatically only by passing the user terminal 300 or an IC card over the user identification unit 220. Further, a network can be identified automatically and eventually, the user can be determined to be in a specific organization (a company, a hospital, or a school, for example). In this way, each time the user uses a toilet, the user can be identified simply without the need of inputting information for identifying the user or information for determining that the user is in the specific organization.

The user identification unit 220 may include a measurement part 221. The measurement part 221 measures the weight of a user (Kg) received by a toilet seat in the case of a western-style toilet, for example. The measurement part 221 stores information about the measured weight of each user (hereinafter called "weight information") into the storage 250. The user identification unit 220 identifies the user based on the weight information to generate user identification information. As another example, the user identification unit 220 may identify the user by inclusion of a face authentication sensor for face authentication, a posture detection sensor for posture detection, heart rate measurement means for measurement of the heart rate of the user, blood pressure measurement means for measurement of the blood pressure of the user, body fat measurement means for measurement of the body fat of the user, and muscle mass measurement means for measurement of the muscle mass of the user.

The aforementioned pieces of user identification information may be transmitted to the server 100 together with water temperature information, voltage information, user identification information, illuminance information, and imaging information forming a set with the aforementioned pieces of user identification information. Alternatively, transmission of the aforementioned pieces of user identification information may be timed to occur when these pieces of user identification information are identified. To transmit these pieces of user identification information to the server 100, the user identification unit 200 transmits these pieces of information to the transmission part 242 via the controller 230. By doing so, a user can be identified automatically in the course of normal urination. In this way, each time the user uses a toilet, the user can be identified simply without the need of inputting information for identifying the user.

The controller 230 is a processor having the function of controlling each part of the measurement device 200. The controller 230 may include input means (not shown in the drawings) allowing a user to select start of each measurement relating to excreted urine manually. The controller 230 transmits input of start of measurement given by using the input means to the measurement unit 210.

The communication unit 240 includes a receiving part 241 and a transmission part 242. The communication unit 240 has the function of making communication with the server 100 and each user terminal 200 via the network 400. This communication can be either wire communication or wireless communication (a communication system such as Wi-Fi (Wireless Fidelity), BLE (Bluetooth low energy), or ZigBee, for example). Further, any communication protocol can be used as long as mutual communication can be established.

The receiving part 241 has the function of receiving control data, etc. from each server 100 and each user terminal 300 via the network 400 under control by the controller 230, and transmitting the received control data, etc. to the controller 120. More specifically, the receiving part 241 receives user information (ID information, for example) from the server 100 to be stored in the storage 130 for control over the user identification unit 220, dynamic parameter data, etc. required for measurement and image capturing by the measurement unit 210 and identification by the user identification unit 220. Then, the receiving part 241 transmits the received information to the controller 230.

The transmission part 242 has the function of transmitting measurement data, etc. to the server 100 and each user terminal 300 via the network 400 under control by the controller 230. Specific examples of information to be transmitted from the transmission part 242 to the server 100 or each user terminal 300 include water temperature information, voltage information, user identification information (including measurement information), illuminance information, and imaging information. The following describes examples of timing of transmission from transmission part 242: (1) immediately after measurement (using transmission of measurement data from the measurement unit 210 as a trigger, for example); (2) cyclically (at regular time intervals determined in consideration of life rhythm of a user or the capacity of the storage 259, for example); and (3) at a time when a threshold set for the storage capacity of a storage 250 is reached.

The storage 250 has the function of storing various programs, various types of data, and various parameters required for operating the measurement device 200. More specifically, the storage 250 stores user information, and parameters required for the operations of the measurement unit 210, the user identification unit 220, the controller 230, and the communication unit 240, for example. The storage 250 is typically realized by every type of recording medium such as an HDD (hard disc drive), an SSD (solid state drive), and a flash memory (an SD (secure digital) memory card), for example.

The configuration of the measurement device 200 is as described above.

The configuration of the user terminal 300 will be described next. As shown in Fig. 2, the user terminal 300 includes a communication unit 310, the controller 320, the display unit 330, and a storage 340. Each part of the user terminal 300 may be incorporated in the health monitoring application or in a circuit of the user terminal 300.

The communication unit 310 includes a receiving part 311 and a transmission part 312. The communication unit 310 has the function of making communication with the server 100 and each measurement device 200 via the network 400. This communication can be either wire communication or wireless communication. Further, any communication protocol can be used as long as mutual communication can be established.

The receiving part 311 has the function of receiving display data, etc. from each server 100 and each measurement device 200 via the network 400 under control by the controller 320, and transmitting the received display data, etc. to the controller 320. More specifically, the receiving part 311 receives display information containing a result of test on urine from the server 100, for example. The receiving part 311 further receives user information (ID information, for example) to be stored in the storage 130 for control over the user identification unit 220, and dynamic parameter data, etc. required for measurement and image capturing by the measurement unit 210 and identification by the user identification unit 220. The receiving part 311 transmits the received information to the controller 230.

The transmission part 312 has the function of transmitting input information input by a user through the display unit 330, user identification information such as QR code (registered trademark) information, etc. to the server 100 and each measurement device 200 via the network 400 under control by the controller 320.

The controller 320 is a processor having the function of controlling each part of the user terminal 300. In response to transmission of an input result from the display unit 330 or transmission of an estimation result from the estimation part 124, the controller 320 generates display data for display of the transmitted result in a text, table, or graph format on the display unit 330. To transmit the generated display data to the user terminal 300, the controller 120 transmits this data to the transmission part 112.

The display unit 330 has the function of displaying display data, etc. received from the server 100 or the measurement device 200. More specifically, as shown in Fig. 3, the display unit 330 displays display data indicating a measured value about measured excreted urine, a measurement result for example indicating whether the measured value is normal or abnormal, an assay result about an assayed urine component, and a monitoring result such as an estimation result indicating whether being positive or negative for an estimated disease in a text, table, or graph format, for example. These results may be displayed on a basis designated by a user such as a daily basis, a weekly basis, or a monthly basis, for example. The display unit 330 may include input means for a user. For example, the display unit 330 may cause the user to input user identification information (name, age, sex, height, or weight, for example) with the input means.

The storage 340 has the function of storing various programs, various types of data, and various parameters required for the operation of the user terminal 300. More specifically, the storage 340 stores user identification information and parameters required for the operations of the communication unit 310, the controller 320, the display unit 330, and the storage 340. The storage 250 is typically realized by every type of recording medium such as an HDD (hard disc drive), an SSD (solid state drive), and a flash memory (an SD (secure digital) memory card), for example. The configuration of the user terminal 300 is as described above.

Fig. 3 schematically shows an exemplary outline of the health monitoring system 100. According to this exemplary configuration, the health monitoring system is used while the measurement device 200 is installed on a western-style toilet. As long as a toilet to be used for installation has a puddle of water for cleaning and drainage, the style of the toilet is not limited to a western style but it can be any type such as a Japanese style, for example. As shown in Fig. 3, a part of the measurement device 200 (measurement unit 210, for example) for measuring information about a puddle of water and information about water containing excreted urine is installed on a device to be dipped in the puddle of water in a toilet bowl. The other parts (user identification unit 220, controller 230, and communication unit 240, for example) not required to be dipped in the puddle of water may be installed on a different device and this device may be installed on a tank, for example. It is preferable that the device for installation of the user identification unit 220 be a device that can be arranged at a position where the user terminal 300 can be passed over the device so as to allow the device to read QR code information output from the user terminal 300 belonging to a user and information output from an IC card belonging to the user. This makes it possible to identify a user and then make measurement without requiring the user to input user identification information to the measurement device 200 for each use of the measurement device 200.

The internal configuration of the measurement unit 210 forming the measurement device 200 will be described next.

### <First Embodiment>

Fig. 5 schematically shows an exemplary internal configuration of the measurement unit 210 according to the first embodiment. Specifically, Fig. 5 shows an exemplary configuration related to the film of the imaging part 212 configuring the measurement unit 210. As shown in Fig. 5, the imaging part 212 includes two reels in which the film is stretched from one of the reels and wound on the other reel, and rotates the reels automatically each time the measurement is started or each time the measurement is finished so that the film can be sequentially fed and dipped into a puddle of water into which the excreted urine has flowed.

Specifically, an example of a roll type film of the imaging part 212 arranges a reel 10a on which an unused film 30a is wound and a reel 20a for winding up a used film 30a in the measurement unit 210. The film 30a is stretched from the reel 20b and wound around the reel 20a so that the film 30a is sequentially fed and dipped into the puddle of water or water containing excreted urine. The imaging part 212 causes color reaction of the reagent placed on the dipped film and takes an image of the reaction by imaging means (not shown) of the imaging part 212. In addition to the example of the roll type shown in Fig. 5, as an example of the strip type, a film may be taken out one by one, dipped in the puddle of water or water containing excreted urine to make a reagent placed on the film produce color reaction. Accordingly, the user need not replace the film in each time of the measurement, eliminating the efforts of replacement to provide an easy-to-use measurement device 200.

### <Second Embodiment>

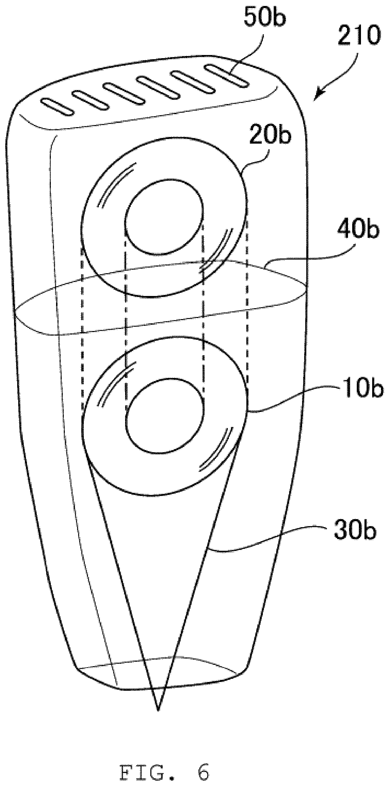

Fig. 6 schematically shows an exemplary internal configuration of the measurement unit 210 according to the first embodiment. As shown in Fig. 6, the measurement unit 210 may be divided into two layers, i.e., an upper part and a lower part with reference to a partition 40 such that the upper part (the side on which the reel 20b is provided where the used film has been wound on the reel 20b) with respect to the partition 40b is set as a discarding portion, and the lower part (the side on which the reel 10b is provided where the unused film is wound on the reel 10b) with respect to the partition 40b is set as a using portion. The discarding portion may be employed as a storage space for discarding the used water-soluble film by soving the film in the puddle of water in the toilet.

Specifically, for example, when the used film flushes (when water in the toilet flushes into the measurement unit 210 during washing of the toilet where the excreted urine has washed out with water), a part of the water may be introduced into the discarding portion to wash out the film to be discarded each time the water flushes. Also, since the discarding portion stores the used film, it is necessary to make the discarding portion as an environment difficult for bacteria to reproduce. Accordingly, specifically, for example, the discarding portion may contain an agent such as a surfactant or it may be sterilized or antibacterial, or it may be kept in a vacuum to prevent propagation. The configuration of the film 30b, the reel 20a, and the reel 10b may be a roll type as shown in the first embodiment, or an automatic cartridge type in which after the film of the reel 10b is completely used, the partition 40b is automatically opened so that the film is moved to the upper discarding portion to be stored. Another reel (not shown) on which the unused film has been wound, which reel is held in the measurement device, is then placed on the reel 10b of the using portion, thereby making the film new. Accordingly, when the measurement unit 210 is used for a long period of time, the measurement unit 210 can easily maintain the hygienic state and continue to be used.

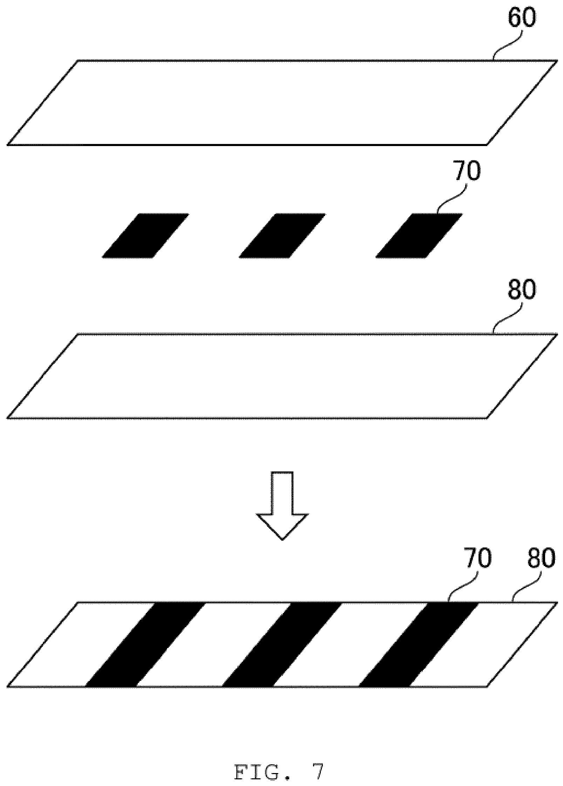

The configuration of the film forming the imaging part 212 and that of a reagent placed on the film will be described next. Fig. 7 schematically shows an exemplary configuration of the film forming the imaging part 212 and that of the reagent. As shown in Fig. 7, by using a top film 60 for protection of a surface of the reagent and a support film 80 for holding the reagent thereon (as a support for the reagent), the film including the top film 60, the support film 80, and the reagent caught between the top film 60 and the support film 80 can be formed as a part of the imaging part 212. The top film 60 can be handled by either of the following ways: (1) The top film 60 is made of a water-soluble film and is dissolved during measurement; and (2) A mechanism for peeling the top film 60 is incorporated in the measurement unit 210 and the top film 60 is peeled immediately before measurement. By employing the way (1) or (2), the reagent can be protected immediately before measurement to prevent degradation of the reagent. Alternatively, the top film 60 may be omitted and the following way (3) may be employed: (3) The reel on which the unused film of the measurement unit 210 has been wound is stored in a highly confidential space. This minimizes the amount of air to which the film is to be exposed immediately before measurement. This can also prevent degradation of the reagent.

### <Data>

Exemplary data configurations of various DBs stored in the storage 130 of this embodiment are described here using Fig. 8. Each of these DBs is not always required to be stored only in the storage 130 of the server 100 but can be stored in the storage 250 of the measurement device 200 or in the storage 330 of the user terminal 300. If appropriate, these data configurations can certainly be changed in a manner that depends on the functional configuration of the server 100 or a process performed by the server 100, for example.

Referring first to a toilet information DB, the toilet information DB is a DB storing information about a toilet. For example, the toilet information DB contains information including the model number of the toilet, a water amount (the water level, mass, volume, etc. of a puddle of water), water temperature (water temperature information about the puddle of water), whether the toilet has been cleaned or not, an installation place (information about latitude and longitude, an address, a building name, etc.), and time of start of use (time when use of the toilet is started). The toilet information DB may additionally contain information about toilet environment (not shown in the drawings) such as amount information about a cleaner, etc. or component information about the cleaner, etc. The toilet information DB holds a record in units of toilets. Information linked with the model number of a toilet (shape information about a bowl of the toilet and water amount information about the toilet, etc.) may be held in the toilet information DB. Alternatively, this information may not be stored in the toilet information DB but may be acquired by search conducted through a network such as the Internet each time the need for the acquisition arises.

Referring next to a threshold DB, the threshold DB is a DB storing a threshold as a criterion for determination as to whether a measurement result is positive or negative, or whether the measurement result is normal or abnormal, for example. For example, the threshold DB contains information including a measurement item, a threshold (an absolute threshold) for each measurement item (a reference value functioning as an absolute index for each measurement item), and a threshold for each measurement item (prepared for each user) (a reference value for each measurement item functioning as an index personalized for each user).

Referring next to a measurement and test result DB, the measurement and test result DB is a DB storing a measurement result and a test result about each user. For example, the measurement and test result DB contains information including a user ID (user identification information), a measurement item, a measured value, a test item, a test result (an assay result or an estimation result), time and date of measurement (year, month, and day, or hour, minute, and second) and time and date of test (year, month, and day, or hour, minute, and second).

Referring next to a dictionary data DB, the dictionary data DB is a DB storing dictionary data. For example, the dictionary data DB contains information including a measured value and a test result (an assay result or an estimation result). The dictionary data DB functions as what is called teacher data for machine learning and is used for identifying a feature vector generated using a measured value. The dictionary data stored in the dictionary data DB may be defined by and stored in a configuration file. Using the configuration file is considered to increase the speed of reading the dictionary data and the speed of updating process, compared to using a DB.

Referring next to a user DB, the user DB is a DB storing information for uniquely identifying a user. For example, the user DB contains information including a user ID (uniquely assigned information in alphanumeric characters), the name, sex, height, and weight of a user, mass information measured by the measurement device 200, and a toilet ID of one or more toilets associated with the user. The data configurations of the various DBs are as described above.

An exemplary data configuration containing association between a result of measurement or assay obtained by the health monitoring system 500 and information about a disease, etc. will be described next using Fig. 10. Fig. 10 is a data conceptual view showing this association. For example, by using an albumin component in excreted urine as input information, the imaging part 212 determines how color appears as a result of color reaction of the film having reacted with a reagent, etc. by an immunochromato method. Based on this color appearance, an albumin concentration in the urine is assayed and it is determined whether or not a result of this assay exceeds a corresponding threshold, for example. Based on a result of this determination, estimation is made as to whether a user is positive or negative for diabetes.

### <Operation>

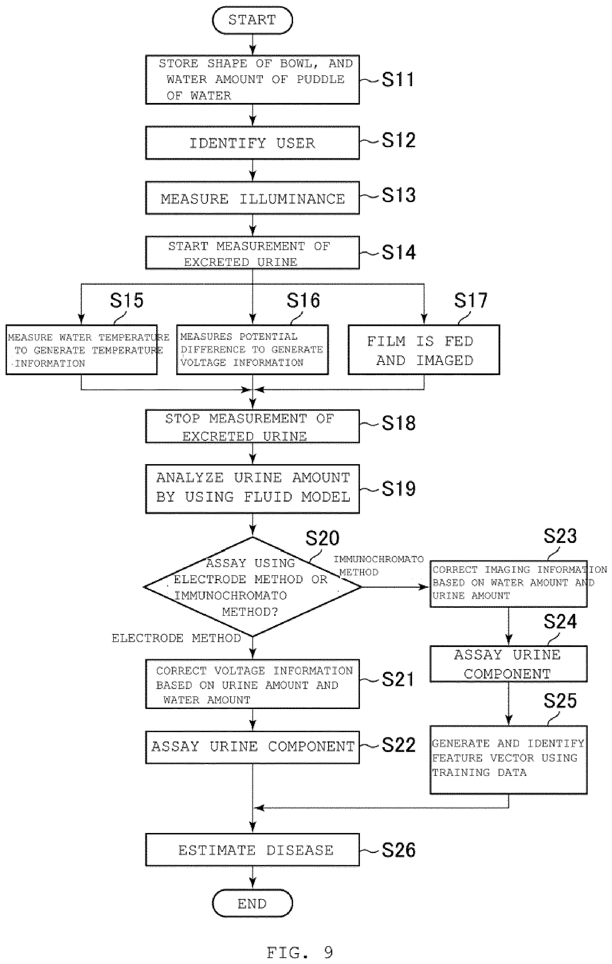

Fig. 9 is a flowchart showing an exemplary process performed by the health monitoring system 100.

As default setting or for each time measurement, shape information about a toilet bowl, and water amount information and water temperature information about a puddle of water are stored in the storage 130 (step S11). The user identification unit 220 identifies a user using an IC card or the user terminal 300, for example (step S12). After this step, the measurement unit 212 may measure the water temperature of the puddle of water once (not shown in the drawings). The illuminance sensor part 213 measures the illuminance of a surface of the film (step S13). If start of measurement is transmitted from the user manually through the input means of the controller 230, the measurement unit 210 starts each measurement (step S14). This step can be omitted if the electrode part 211, the imaging part 212, and the temperature measurement part 214 are to start measurements automatically.

If measured temperature reaches a predetermined threshold, for example, to start measurement automatically or manually, the temperature measurement part 214 measures the temperature of the puddle of water or that of water containing excreted urine to generate water temperature information (step S15). If a measured potential difference reaches a predetermined threshold, for example, to start measurement automatically or manually, the electrode part 211 measures a potential difference between the electrodes to generate voltage information (step S16). If the measurement starts automatically or manually, the imaging part 212 feeds the film so as to dip the sample pad part of the film into the puddle of water or water containing excreted urine (step S17).

If measured temperature reaches a predetermined threshold, for example, the temperature measurement part 214 stops measurement automatically. If a measured potential difference reaches a predetermined threshold, for example, the electrode part 211 stops measurement automatically (step S18).

The analysis part 121 analyzes a fluid to analyze (calculate) a urine amount by using a fluid model formed by modeling a fluid flowing around the measurement unit 210 and based on the shape information, the water amount information, the water temperature information, etc. (step S19). If a value is measured by assay using an electrode method (if an electrode method is selected in step S20), the correction part 122 calculates a degree of dilution based on the analyzed urine amount information and water amount information to correct voltage information based on the calculated degree of dilution (step S21). The assay part 123 assays a urine component based on the voltage information (as corrected) (step S22).

If a value is measured by assay using an immunochromato method (if an immunochromato method is selected in step S20), the correction part 122 calculates a degree of dilution based on the analyzed urine amount information and water amount information to correct imaging information based on the calculated degree of dilution (step S23). In this step, the correction part 122 may correct imaging information based on illuminance information in addition to the calculated degree of dilution. The assay part 123 assays a urine component based on the imaging information (as corrected) (step S24). The assay part 123 generates a feature vector based on a result of the assay and identifies the generated feature vector using training data (dictionary data) (step S25). The estimation part 124 estimates a disease of the user based on excreted urine information about the analyzed excreted urine (the assayed urine component, for example) (step S26).

### (Other Issues)

The health monitoring system according to this invention may work in conjunction with a medical institution, etc. to be usable as a part of telemedicine. For example, the user DB stored in the storage 130 may contain information about a medical institution, a doctor, etc. caring each user. When the measurement and test result DB is updated, for example, a measured value and data about a test result in this DB may be transmitted to the aforementioned medical institution, etc. Based on the transmitted data, even if a patient is at home, the doctor, etc. can given a diagnosis, advice, and the like about a health from a remote place to the patient.

The health monitoring system according to this invention is also usable for remote observation for the administration of medicine for example by a doctor, a pharmacist, or a pharmaceutical company (to check the administration of prescribed medicine), check for drug metabolism (to check the effectiveness of prescribed medicine), service of delivering medicine from a pharmacy conforming to a health condition or according to a prescription from a doctor, and check for the health of a family member in a remote place, for example. Further, the health monitoring system according to this invention generates time-series vital data based on measurement and test result information stored in the storage 130. In conjunction with a system in a pharmaceutical company or a health insurance association, this vital data can be used for data marketing business. In conjunction with a system in an insurance company or a health insurance association, the vital data can also be used for a simulation conducted to see how medical expenses can be reduced.

The health monitoring system according to this invention also becomes usable for service of providing more individual and more specific advice on health and beauty care by associating the aforementioned generated vital data with a lifelog recorded daily by a wearable appliance working in conjunction with the health monitoring system. Associating the vital data and the lifelog also makes it possible to make the health monitoring system usable for making a model showing the health condition of a person and showing how this person lives a life, for example.

As an example, if the vital data is associated with a lifelog about a meal, an inadequate nutrient, etc. is extracted from the vital data, the extracted nutrient is displayed on the display unit 330 of the user terminal 300, and a meal menu (including information about a food material such as a vegetable to be taken) and a supplement can be suggested based on the extracted nutrient by displaying such a meal menu and a supplement on the display unit 330 of the user terminal 300. Further, the vital data can be categorized into types and a supplement to make up for a nutrient inadequate in a body can be suggested according to each type. Such service is to be provided not only to general households or individuals but is also applicable for health management of athletes, for example. Such service is also applicable in the field of beauty care. In this case, personalized cosmetics can be suggested particularly to a user assumed to have trouble with his or her skin or hair.

By associating the vital data not only with a lifelog but also with a result of genome analysis, the health monitoring system according to this invention becomes usable for making a model showing a genome a person has and showing how this person lives a life, for example.

Information about a health condition expected from the aforementioned models may be provided to an insurance company, etc. In this way, the aforementioned pieces of information about the models become usable for the insurance company, etc. to examine or decide the probability of having insurance or an insurance fee based on the expected information.

Each functional part of each of the server 100, the measurement device 200, and the user terminal 300 may be realized by a logic circuit (hardware) or a dedicated circuit formed in an integrated circuit (an IC (integrated circuit) chip or an LSI (large scale integration)), for example. Alternatively, each functional part may be realized by software using a CPU (central processing unit) and a memory. Each functional part may be realized by one or a plurality of integrated circuits. Alternatively, the functions of a plurality of functional parts may be realized by one integrated circuit. An LSI may be called a VLSI, a super LSI, or an ultra LSI. These names result from a difference in integration level. The "circuit" mentioned herein may have a meaning as digital processing performed by a computer, specifically, functional processing by software. This circuit may alternatively be realized by a circuit that can be rebuilt such as an FPGA (field programmable gate away), for example.

If each functional part of each of the server 100, the measurement device 200, and the user terminal 300 is realized by software, each functional part of the server 100, the measurement device 200, or the user terminal 300 includes: a CPU that executes an order given by a display information generation program as software for realizing each function; a ROM (read only memory) or a storage (each of them will be called a "recording medium") storing the aforementioned health monitoring program and various types of data in such a manner that the health monitoring program and such data are readable by a computer (or the CPU); and a RAM that expands the health monitoring program, for example. The computer (or the CPU) reads the health monitoring program from the recording medium and executes the read program, thereby achieving the object of this invention. A "non-transitory tangible medium" such as a tape, a disk, a card, a semiconductor memory, or a programmable logic circuit is usable as the aforementioned recording medium, for example. The health monitoring program may be supplied to the computer via any transmission medium (such as a communication network or a broadcast wave) that can transmit the health monitoring program. This invention can also be embodied by electronic transmission of the health monitoring program to be realized in the form of a data signal embedded in a carrier wave.

The aforementioned health monitoring program can be implemented using a script language such as ActionScript or JavaScript (registered trademark), an object-orientated programming language such as Objective-C or Java (registered trademark), or a markup language such as HTML5, for example.

## Claims

1. A health monitoring system comprising:
a storage (130) that stores shape information about a bowl of a toilet and water amount information about a puddle of water in said bowl;
a measurement unit (210) for measuring fluid information about a fluid in the puddle of water into which urine excreted by a user of the toilet has flowed, the fluid information contains water temperature information generated as a result of measurement of the water temperature of at least one of a puddle of water and a puddle of water into which the excreted urine has flowed;
an analysis part (121) configured to analyse the excreted urine by assaying a fluid model formed by modeling the fluid based on the fluid information and at least one of the shape information and the water amount information and further configured to generate excreted urine information;
an imaging part (212) that includes a film that changes in color in response to a component in the puddle of water into which the excreted urine has flowed, the imaging part being configured to expose said film to the puddle of water into which the excreted urine has flowed, and imaging means for generating imaging information by capturing an image of the film;
a correction part (122) configured to correct the imaging information based on water amount information and the excreted urine information containing the amount of the excreted urine;
an assay part (123) configured to assay a urine component based on corrected imaging information;
an estimation part (124) configured to estimate a disease of the user based on the urine component.

2. The health monitoring system according to claim 1, **characterized in that**
the measurement unit (210) is further adapted to generate voltage information by measuring a potential difference between two electrodes dipped in the puddle of water or the puddle of water into which the excreted urine has flowed,
the correction part (122) is further adapted to correct the voltage information based on the water amount information and the excreted urine information containing the amount of the excreted urine; and **in that**
the assay part (123) is further adapted to assay the urine component based on corrected voltage information.

3. The health monitoring system according to claim 2, **characterized in that** if at least one of the water temperature information and the voltage information reaches a predetermined threshold, the measurement unit (210) is configured to start or to finish at least one of measurement of the water temperature of the puddle of water into which the excreted urine has flowed and measurement of the potential difference.

4. The health monitoring system according to claim 3, **characterized in that**
the measurement unit (210) further includes an illuminance sensor part for measuring an illuminance,
the storage (130) stores illuminance information for the toilet, and **in that**
the correction part (122) is configured to correct the imaging information based on the illuminance information.

5. The health monitoring system according to claim 3 or 4, **characterized in that** the estimation part (124) is further configured to generate a feature vector based on the imaging information, to identify the generated feature vector using training data, and to estimate a disease based on the identified feature vector.

6. The health monitoring system according to any one of claims 1 to 5, **characterized by** further comprising a user identification unit (220) for identifying the user based on user identification information output from a terminal or an IC card belonging to the user, wherein the estimation part (124) is configured to estimate a disease of the user based on a result of the identification.

7. The health monitoring system according to claim 6, wherein
the user identification unit (220) further includes a measurement part that generates weight information by measuring the weight of the user received by a seat of the toilet when the user uses the seat, and
the user identification unit (220) is configured to identify the user based on the weight information.

8. The health monitoring system according to claim 1, wherein
the imaging part (212) includes two reels in which the film is stretched from one of the reels and wound by another one of the reels, and the reels are automatically rotated each time the measurement is started or each time the measurement is finished so that the film is sequentially fed and dipped into the puddle of water into which the excreted urine has flowed, and
the film is configured such that reagent is caught between a top film and a support film.

9. A health monitoring method comprising:
a storage step of storing shape information about a bowl of a toilet and water amount information about a puddle of water in said bowl;
a measurement step of measuring fluid information about a fluid in the puddle of water into which urine excreted by a user of the toilet has flowed, the fluid information contains water temperature information generated as a result of measurement of the water temperature of at least one of a puddle of water and a puddle of water into which the excreted urine has flowed;
an analysis step of analyzing the excreted urine by assaying a fluid model formed by modeling a region where the fluid flows based on the fluid information and at least one of the shape information and the water amount information and generating excreted urine information;
an imaging step of generating imaging information by capturing an image of a film that changes in color in response to a component in the puddle of water into which the excreted urine has flowed, the image being captured after said film was exposed to the puddle of water into which the excreted urine has flowed;
a correction step of correcting the imaging information based on water amount information and the excreted urine information containing the amount of the excreted urine;
an assay step of assaying a urine component based on corrected imaging information;
an estimation step of estimating a disease of the user based on the urine component.

10. A health monitoring program executed on the system of claim 1, comprising:
a storage function of storing shape information about a bowl of each toilet and water amount information about a puddle of water;
a measurement function of measuring fluid information about a fluid in the puddle of water into which urine excreted by a user of the toilet has flowed, the fluid information contains water temperature information generated as a result of measurement of water temperature of at least one of a puddle of water and a puddle of water into which the excreted urine has flowed;
an analysis function of analyzing the excreted urine by assaying a fluid model formed by modeling a region where the fluid flows based on the fluid information and at least one of the shape information and the water amount information and generating excreted urine information;
an imaging function of generating imaging information by capturing an image of a film that changes in color in response to a component in the puddle of water into which the excreted urine has flowed, the imaging information being generated by capturing an image after said film was exposed to the puddle of water into which the excreted urine has flowed;
a correction function of correcting the imaging information based on water amount information and the excreted urine information containing the amount of the excreted urine;
an assay function of assaying a urine component based on corrected imaging information; and
an estimation function of estimating a disease of the user based on the urine component.

## Patentansprüche

1. Gesundheitsüberwachungssystem, aufweisend:
einen Speicher (130), in dem Forminformationen über eine Schüssel einer Toilette und Wassermengeninformationen über einen Bestand an Wasser in der Schüssel gespeichert sind;
eine Messeinheit (210) zum Messen von Fluidinformationen über ein Fluid in dem Bestand an Wasser, in den von einem Benutzer ausgeschiedener Urin eingeströmt ist, wobei die Fluidinformationen Wassertemperaturinformationen enthalten, die infolge einer Messung der Wassertemperatur von einem Bestand an Wasser und/oder einem Bestand an Wasser, in den der ausgeschiedene Urin eingeströmt ist, erzeugt werden;
einen Analyseabschnitt (121), der dafür ausgelegt ist, den ausgeschiedenen Urin zu analysieren, indem ein Fluidmodell untersucht wird, das gebildet wird, indem das Fluid auf der Grundlage der Fluidinformationen sowie der Forminformationen und/oder der Wassermengeninformationen modellhaft nachgebildet wird, und der darüber hinaus dafür ausgelegt ist, Informationen über den ausgeschiedenen Urin zu erzeugen;
einen Bildgebungsabschnitt (212), der einen Film, der sich als Reaktion auf eine Komponente in dem Bestand an Wasser, in den der ausgeschiedene Urin eingeströmt ist, in der Farbe ändert, wobei der Bildgebungsabschnitt dafür ausgelegt ist, den Film dem Bestand an Wasser auszusetzen, in den der ausgeschiedene Urin eingeströmt ist, und Bildgebungsmittel zum Erzeugen von Bildgebungsinformationen umfasst, indem ein Bild des Films aufgenommen wird;
einen Korrekturabschnitt (122), der dafür ausgelegt ist, die Bildgebungsinformationen auf der Grundlage der Wassermengeninformationen und der Informationen über den ausgeschiedenen Urin, welche die Menge des ausgeschiedenen Urins enthalten, zu korrigieren;
einen Untersuchungsabschnitt (123), der dafür ausgelegt ist, eine Urinkomponente auf der Grundlage von korrigierten Bildgebungsinformationen zu untersuchen;
einen Abschätzungsabschnitt (124), der dafür ausgelegt ist, eine Krankheit des Benutzers auf der Grundlage der Urinkomponente abzuschätzen.

2. Gesundheitsüberwachungssystem nach Anspruch 1, **dadurch gekennzeichnet, dass**
die Messeinheit (210) darüber hinaus dazu angepasst ist, Spannungsinformationen zu erzeugen, indem ein Potentialunterschied zwischen zwei Elektroden gemessen wird, die in den Bestand an Wasser oder in den Bestand an Wasser, in den der ausgeschiedene Urin eingeströmt ist, eingetaucht sind,
der Korrekturabschnitt (122) darüber hinaus dazu angepasst ist, die Spannungsinformationen auf der Grundlage der Wassermengeninformationen und der Informationen über den ausgeschiedenen Urin, welche die Menge des ausgeschiedenen Urins enthalten, zu korrigieren; und dass
der Untersuchungsabschnitt (123) darüber hinaus dazu angepasst ist, die Urinkomponente auf der Grundlage von korrigierten Spannungsinformationen zu untersuchen.

3. Gesundheitsüberwachungssystem nach Anspruch 2, **dadurch gekennzeichnet, dass**,
wenn die Wassertemperaturinformationen und/oder die Spannungsinformationen einen vorbestimmten Schwellenwert erreichen, die Messeinheit (210) dafür ausgelegt ist, die Messung der Wassertemperatur des Bestands an Wasser, in den der ausgeschiedene Urin eingeströmt ist, und/oder die Messung des Potentialunterschieds zu starten oder zu beenden.

4. Gesundheitsüberwachungssystem nach Anspruch 3, **dadurch gekennzeichnet, dass**
die Messeinheit (210) darüber hinaus einen Beleuchtungsstärken-Sensorabschnitt zum Messen einer Beleuchtungsstärke umfasst,
wobei in dem Speicher (130) Beleuchtungsstärkeinformationen zu der Toilette gespeichert sind, und dass
der Korrekturabschnitt (122) dafür ausgelegt ist, die Bildgebungsinformationen auf der Grundlage der Beleuchtungsstärkeinformationen zu korrigieren.

5. Gesundheitsüberwachungssystem nach Anspruch 3 oder 4, **dadurch gekennzeichnet, dass**
der Abschätzungsabschnitt (124) darüber hinaus dafür ausgelegt ist, einen Eigenschaftsvektor auf der Grundlage der Bildgebungsinformationen zu erzeugen, den erzeugten Eigenschaftsvektor mithilfe von Trainingsdaten zu erkennen und eine Krankheit auf der Grundlage des erkannten Eigenschaftsvektors abzuschätzen.

6. Gesundheitsüberwachungssystem nach einem der Ansprüche 1 bis 5, **dadurch gekennzeichnet, dass** es darüber hinaus eine Benutzeridentifikationseinheit (220) zum Erkennen des Benutzers auf der Grundlage von Benutzeridentifikationsinformationen aufweist, die von einem Endgerät oder einer IC-Karte ausgegeben werden, das bzw. die dem Benutzer gehört, wobei der Abschätzungsabschnitt (124) dafür ausgelegt ist, eine Krankheit des Benutzers auf der Grundlage eines Ergebnisses der Erkennung abzuschätzen.

7. Gesundheitsüberwachungssystem nach Anspruch 6, wobei
die Benutzeridentifikationseinheit (220) darüber hinaus einen Messabschnitt umfasst, der Gewichtsinformationen erzeugt, indem das Gewicht des Benutzers gemessen wird, das von einem Sitz der Toilette aufgenommen wird, wenn der Benutzer den Sitz benutzt, und
die Benutzeridentifikationseinheit (220) dafür ausgelegt ist, den Benutzer auf der Grundlage der Gewichtsinformationen zu erkennen.

8. Gesundheitsüberwachungssystem nach Anspruch 1, wobei
der Bildgebungsabschnitt (212) zwei Rollen umfasst, in denen der Film von einer der Rollen abgezogen und von einer anderen der Rollen aufgewickelt wird, und die Rollen immer dann automatisch gedreht werden, wenn die Messung gestartet wird, und immer dann, wenn die Messung beendet wird, so dass der Film sequentiell zugeführt und in den Bestand an Wasser eingetaucht wird, in den der ausgeschiedene Urin eingeströmt ist, und
der Film so ausgelegt ist, dass ein Reagenzstoff zwischen einem oberen Film und einem Trägerfilm eingeschlossen ist.

9. Gesundheitsüberwachungsverfahren, umfassend:
einen Speicherschritt des Speicherns von Forminformationen über eine Schüssel einer Toilette und von Wassermengeninformationen über einen Bestand an Wasser in der Schüssel;
einen Messschritt des Messens von Fluidinformationen über ein Fluid in dem Bestand an Wasser, in den von einem Benutzers der Toilette ausgeschiedener Urin eingeströmt ist, wobei die Fluidinformationen Wassertemperaturinformationen enthalten, die infolge einer Messung der Wassertemperatur von einem Bestand an Wasser und/oder einem Bestand an Wasser, in den der ausgeschiedene Urin eingeströmt ist, erzeugt werden;
einen Analyseschritt des Analysierens des ausgeschiedenen Urins, indem ein Fluidmodell untersucht wird, das gebildet wird, indem ein Bereich, wo das Fluid strömt, auf der Grundlage der Fluidinformationen sowie der Forminformationen und/oder der Wassermengeninformationen modellhaft nachgebildet wird, und des Erzeugens von Informationen über den ausgeschiedenen Urin;
einen Bildgebungsschritt des Erzeugens von Bildgebungsinformationen, indem ein Bild eines Films aufgenommen wird, der sich als Reaktion auf eine Komponente in dem Bestand an Wasser, in den der ausgeschiedene Urin eingeströmt ist, in der Farbe ändert, wobei das Bild aufgenommen wird, nachdem der Film dem Bestand an Wasser ausgesetzt war, in den der ausgeschiedene Urin eingeströmt ist;
einen Korrekturschritt des Korrigierens der Bildgebungsinformationen auf der Grundlage der Wassermengeninformationen und der Informationen über den ausgeschiedenen Urin, welche die Menge des ausgeschiedenen Urins enthalten;
einen Untersuchungsschritt des Untersuchens einer Urinkomponente auf der Grundlage von korrigierten Bildgebungsinformationen;
einen Abschätzungsschritt des Abschätzens einer Krankheit des Benutzers auf der Grundlage der Urinkomponente.

10. Gesundheitsüberwachungsprogramm, das auf dem System nach Anspruch 1 ausgeführt wird, umfassend:
eine Speicherfunktion zum Speichern von Forminformationen über eine Schüssel jeder Toilette und von Wassermengeninformationen über einen Bestand an Wasser;
eine Messfunktion zum Messen von Fluidinformationen über ein Fluid in dem Bestand an Wasser, in den von einem Benutzer der Toilette ausgeschiedener Urin eingeströmt ist, wobei die Fluidinformationen Wassertemperaturinformationen enthalten, die infolge einer Messung der Wassertemperatur von einem Bestand an Wasser und/oder einem Bestand an Wasser, in den der ausgeschiedene Urin eingeströmt ist, erzeugt werden;
eine Analysefunktion zum Analysieren des ausgeschiedenen Urins, indem ein Fluidmodell untersucht wird, das gebildet wird, indem ein Bereich, wo das Fluid strömt, auf der Grundlage der Fluidinformationen sowie der Forminformationen und/oder der Wassermengeninformationen modellhaft nachgebildet wird, und zum Erzeugen von Informationen über den ausgeschiedenen Urin;
eine Bildgebungsfunktion zum Erzeugen von Bildgebungsinformationen, indem ein Bild eines Films aufgenommen wird, der sich als Reaktion auf eine Komponente in dem Bestand an Wasser, in den der ausgeschiedene Urin eingeströmt ist, in der Farbe ändert, wobei die Bildgebungsinformationen erzeugt werden, indem ein Bild aufgenommen wird, nachdem der Film dem Bestand an Wasser ausgesetzt war, in den der ausgeschiedene Urin eingeströmt ist;
eine Korrekturfunktion zum Korrigieren der Bildgebungsinformationen auf der Grundlage der Wassermengeninformationen und der Informationen über den ausgeschiedenen Urin, welche die Menge des ausgeschiedenen Urins enthalten;
eine Untersuchungsfunktion zum Untersuchen einer Urinkomponente auf der Grundlage von korrigierten Bildgebungsinformationen; und
eine Abschätzungsfunktion zum Abschätzen einer Krankheit des Benutzers auf der Grundlage der Urinkomponente.

## Revendications

1. Système de surveillance de santé comprenant :
une mémoire (130) qui mémorise des informations de forme sur une cuvette d'un W.C. et des informations de quantité d'eau sur un bain d'eau dans ladite cuvette ;
une unité de mesure (210) destinée à mesurer des informations de fluide sur un fluide dans le bain d'eau dans lequel de l'urine excrétée par un utilisateur du W.C. s'est écoulée, les informations de fluide contenant des informations de température d'eau générées comme résultat de mesure de la température d'eau d'au moins un terme parmi un bain d'eau et un bain d'eau dans lequel l'urine excrétée s'est écoulée ;
une partie analyse (121) configurée pour analyser l'urine excrétée en testant un modèle de fluide formé en modélisant le fluide sur la base des informations de fluide et d'au moins un terme parmi les informations de forme et les informations de quantité d'eau et configurée en outre pour générer des informations d'urine excrétée ;
une partie imagerie (212) qui inclut un film qui change de couleur en réponse à une composante dans le bain d'eau dans lequel l'urine excrétée s'est écoulée, la partie imagerie étant configurée pour exposer ledit film au bain d'eau dans lequel l'urine excrétée s'est écoulée, et des moyens d'imagerie destinés à générer des informations d'imagerie en capturant une image du film ;
une partie correction (122) configurée pour corriger les informations d'imagerie sur la base des informations de quantité d'eau et des informations d'urine excrétée contenant la quantité de l'urine excrétée ;
une partie test (123) configurée pour tester une composante d'urine sur la base d'informations d'imagerie corrigées ;
une partie estimation (124) configurée pour estimer une maladie de l'utilisateur sur la base de la composante d'urine.

2. Le système de surveillance de santé selon la revendication 1, **caractérisé en ce que**
l'unité de mesure (210) est en outre apte à générer des informations de tension en mesurant une différence de potentiel entre deux électrodes immergées dans le bain d'eau ou le bain d'eau dans lequel l'urine excrétée s'est écoulée,
la partie correction (122) est en outre apte à corriger les informations de tension sur la base des informations de quantité d'eau et des informations d'urine excrétée contenant la quantité de l'urine excrétée ; et **en ce que**
la partie test (123) en en outre apte à tester la composante d'urine sur la base d'informations de tension corrigées.

3. Le système de surveillance de santé selon la revendication 2, **caractérisé en ce que**
si au moins un terme parmi les informations de température d'eau et les informations de tension atteint un seuil prédéterminé, l'unité de mesure (210) est configurée pour démarrer ou pour terminer au moins un terme parmi la mesure de la température d'eau du bain d'eau dans lequel l'urine excrétée s'est écoulée et la mesure de la différence de potentiel.

4. Le système de surveillance de santé selon la revendication 3, **caractérisé en ce que**
l'unité de mesure (210) inclut en outre une partie capteur d'éclairement destinée à mesurer un éclairement,
la mémoire (130) mémorise des informations d'éclairement pour le W.C., et **en ce que**
la partie correction (122) est configurée pour corriger les informations d'imagerie sur la base des informations d'éclairement.

5. Le système de surveillance de santé selon la revendication 3 ou 4, **caractérisé en ce que**
la partie estimation (124) est en outre configurée pour générer un vecteur de caractéristique sur la base des informations d'imagerie, pour identifier le vecteur de caractéristique généré moyennant des données d'apprentissage, et pour estimer une maladie sur la base du vecteur de caractéristique identifié.

6. Le système de surveillance de santé selon l'une quelconque des revendications 1 à 5, **caractérisé en ce qu'**il comprend en outre une unité d'identification d'utilisateur (220) destinée à identifier l'utilisateur sur la base d'informations d'identification d'utilisateur sorties depuis un terminal ou une carte IC appartenant à l'utilisateur, sachant que la partie estimation (124) est configurée pour estimer une maladie de l'utilisateur sur la base d'un résultat de l'identification.

7. Le système de surveillance de santé selon la revendication 6, sachant que
l'unité d'identification d'utilisateur (220) inclut en outre une partie mesure qui génère des informations de poids en mesurant le poids de l'utilisateur reçu par un siège du W.C. lorsque l'utilisateur utilise le siège, et
l'unité d'identification d'utilisateur (220) est configurée pour identifier l'utilisateur sur la base des informations de poids.

8. Le système de surveillance de santé selon la revendication 1, sachant que
la partie imagerie (212) inclut deux bobines dans lesquelles le film est étiré depuis une des bobines et enroulé par une autre des bobines, et les bobines tournent automatiquement chaque fois que la mesure est démarrée ou chaque fois que la mesure est terminée de telle sorte que le film soit amené séquentiellement et immergé dans le bain d'eau dans lequel l'urine excrétée s'est écoulée, et
le film est configuré de telle sorte que du réactif soit pris entre un film supérieur et un film de support.

9. Procédé de surveillance de santé comprenant :
une étape de mémorisation consistant à mémoriser des informations de forme sur une cuvette d'un W.C. et des informations de quantité d'eau sur un bain d'eau dans ladite cuvette ;
une étape de mesure consistant à mesurer des informations de fluide sur un fluide dans le bain d'eau dans lequel de l'urine excrétée par un utilisateur du W.C. s'est écoulée, les informations de fluide contenant des informations de température d'eau générées comme résultat de mesure de la température d'eau d'au moins un terme parmi un bain d'eau et un bain d'eau dans lequel l'urine excrétée s'est écoulée ;
une étape d'analyse consistant à analyser l'urine excrétée en testant un modèle de fluide formé en modélisant une région où le fluide s'écoule sur la base des informations de fluide et d'au moins un terme parmi les informations de forme et les informations de quantité d'eau et à générer des informations d'urine excrétée ;
une étape d'imagerie consistant à générer des informations d'imagerie en capturant une image d'un film qui change de couleur en réponse à une composante dans le bain d'eau dans lequel l'urine excrétée s'est écoulée, l'image étant capturée après que ledit film a été exposé au bain d'eau dans lequel l'urine excrétée s'est écoulée ;
une étape de correction consistant à corriger les informations d'imagerie sur la base d'informations de quantité d'eau et des informations d'urine excrétée contenant la quantité de l'urine excrétée ;
une étape de test consistant à tester une composante d'urine sur la base d'informations d'imagerie corrigées ;
une étape d'estimation consistant à estimer une maladie de l'utilisateur sur la base de la composante d'urine.

10. Programme de surveillance de santé exécuté sur le système de la revendication 1, comprenant :
une fonction de mémorisation consistant à mémoriser des informations de forme sur une cuvette de chaque W.C. et des informations de quantité d'eau sur un bain d'eau ;
une fonction de mesure consistant à mesurer des informations de fluide sur un fluide dans le bain d'eau dans lequel de l'urine excrétée par un utilisateur du W.C. s'est écoulée, les informations de fluide contenant des informations de température d'eau générées comme résultat de mesure de température d'eau d'au moins un terme parmi un bain d'eau et un bain d'eau dans lequel l'urine excrétée s'est écoulée ;
une fonction d'analyse consistant à analyser l'urine excrétée en testant un modèle de fluide formé en modélisant une région où le fluide s'écoule sur la base des informations de fluide et d'au moins un terme parmi les informations de forme et les informations de quantité d'eau et à générer des informations d'urine excrétée ;
une fonction d'imagerie consistant à générer des informations d'imagerie en capturant une image d'un film qui change de couleur en réponse à une composante dans le bain d'eau dans lequel l'urine excrétée s'est écoulée, les informations d'imagerie étant générées en capturant une image après que ledit film a été exposé au bain d'eau dans lequel l'urine excrétée s'est écoulée ;
une fonction de correction consistant à corriger les informations d'imagerie sur la base d'informations de quantité d'eau et des informations d'urine excrétée contenant la quantité de l'urine excrétée ;
une fonction de test consistant à tester une composante d'urine sur la base d'informations d'imagerie corrigées ; et
une fonction d'estimation consistant à estimer une maladie de l'utilisateur sur la base de la composante d'urine.
